# EUROPÄISCHE PATENTSCHRIFT

(11) **EP 2 499 232 B1**
(45) Veröffentlichungstag und Bekanntmachung des Hinweises auf die Patenterteilung: **30.05.2018**
(21) Anmeldenummer: 10772980.8
(22) Anmeldetag: 20.10.2010
(51) Int. Cl.: C12M 1/12, G01N 1/40, C12M 1/26, B01L 3/00

(54) **VORRICHTUNG UND VERFAHREN ZUR BEHANDLUNG EINES FILTRATIONSMEDIUMS**
DEVICE AND METHOD FOR TREATING A FILTRATION MEDIUM
DISPOSITIF ET PROCÉDÉ POUR TRAITER UN MOYEN DE FILTRATION

(30) Priorität: 12.11.2009 DE 102009052671
(43) Veröffentlichungstag der Anmeldung: 19.09.2012
(73) Patentinhaber: Sartorius Stedim Biotech GmbH, 37079 Göttingen (DE)
(72) Erfinder: PFLANZ, Karl, 37130 Gleichen (DE)
(86) Internationale Anmeldenummer: PCT/EP2010/006416
(87) Internationale Veröffentlichungsnummer: WO 2011/057707

(56) Entgegenhaltungen:
- EP-A1- 1 862 535
- EP-A2- 0 239 058
- DE-A1- 10 054 632
- DE-A1-102008 005 968
- US-A- 5 471 994

## Beschreibung

### Gebiet der Erfindung

Die Erfindung betrifft eine Vorrichtung zur Behandlung eines porösen Filtrationsmediums mit einem Aufnahmeteil, das auf ein Unterteil aufsetzbar ist, wobei eine Außenwandung des Aufnahmeteils außerhalb einer für eine Filtration nutzbaren Fläche des Filtrationsmediums positionierbar ist und ein in dem Aufnahmeteil angeordneter Fixierrand auf einem Rand des Filtrationsmediums positionierbar ist.

Weiterhin betrifft die Erfindung ein Verfahren zur Behandlung eines porösen Filtrationsmediums, bei dem ein Aufnahmeteil einer Vorrichtung auf das in einem Unterteil einer Filtrationsvorrichtung angeordnete und einer flüssigen Probe ausgesetzte Filtrationsmedium aufgesetzt wird, wobei ein in dem Aufnahmeteil angeordneter Fixierrand mit einem Rand des Filtrationsmediums verbunden und mit dem verbundenen Filtrationsmedium von dem Unterteil abgehoben wird.

### Stand der Technik

In der Analytik von Flüssigkeiten und Gasen haben sich verschiedene Behandlungsmethoden etabliert, die poröse Medien, wie Filter und Membranen, einsetzen. Zum Beispiel hat sich das Filtrationsverfahren zur Abreicherung und Konzentrierung von gelösten oder partikulären Inhaltsstoffen etabliert. Diese Konzentrierung ist meist notwendig, da die Konzentrationen der Verunreinigungen zu gering sind, um direkte Auswertungen durchzuführen. Die Filtrationsmethoden dienen als Vorstufe für weitere analytische Methoden, wie optische Auswertungen, sowie für weitere physikalische und chemische Reaktionen zur Signalverstärkung.

Für neuere, sensitivere Analysemethoden, wie die PCR-("Polymerase Chain Reaction") oder Massenspektrometer-Analytik können nur sehr geringe Probevolumina verwendet werden. Um die in vielen Fällen typischen Probevolumina von mehr als 100 ml für eine Konzentrierung filtrieren zu können, werden typischerweise Filtrationsmembranen mit 47 mm oder 25 mm Durchmesser eingesetzt. Auch nach der Filtration, wenn die Inhaltsstoffe oder Partikel auf der Filtrationsmembran konzentriert vorliegen, können die membrangebundenen Partikel aufgrund der Membrangröße nicht direkt der Analytik zugeführt werden. Es ist notwendig, die zurückgehaltenen Inhaltsstoffe in ein Probevolumen zu überführen. Wenn die Sensitivität der Methoden voll ausgeschöpft werden soll, müssen diese Volumina so klein wie möglich sein. Volumina kleiner als 1 ml, bevorzugt 100 µl bis zu 1 µl, sind wünschenswert, da z.B. für die PCR-Analytik typischerweise Volumen von 20 µl bis 1 µl eingesetzt werden und die entsprechenden nicht eingesetzten Proberestmengen zu Lasten der Sensitivität gehen.

Aus der DE 10 2008 005 968 A1 ist eine Nährmedieneinheit und ein Verfahren zur Aufnahme eines Filters aus einer Filtrationsvorrichtung bekannt. Die Nährmedieneinheit besteht dabei aus einem Deckel bzw. einem Aufnahmeteil, das die eigentliche Transfereinheit bildet, und einem mit Nährmedium gefüllten Unterteil. Das als Aufnahmeteil ausgebildete Oberteil weist dabei einen Fixierrand auf, der zur Entnahme des Filtrationsmediums aus der Filtrations- oder Behandlungsvorrichtung mit einem Rand des Filters über eine Klebehaftung verbindbar ist.

Aus der DE 10 2008 005 968 A1 ist weiterhin ein Verfahren zur mikrobiologischen Untersuchung flüssiger Proben bekannt, bei dem ein Deckel bzw. Aufnahmeteil einer Nährmedieneinheit auf einen in einem Unterteil einer Filter- bzw. Behandlungsvorrichtung angeordneten, als Membranfilter ausgebildeten Filter mit einem Fixierrand aufgesetzt wird. Dabei wird der Fixierrand des Aufnahmeteils über eine Haftschicht mit einem Rand des Filters verbunden. Anschließend wird das Aufnahmeteil mit dem Filter von einer Filterunterstützung des Unterteils der Filtervorrichtung abgehoben und auf einer Oberfläche eines in dem Unterteil einer Nährmedieneinheit angeordneten Nährbodens abgelegt, wobei der Deckel bzw. das Aufnahmeteil das schalenförmig ausgebildete Unterteil abdeckt.

Nachteilig bei den bekannten Filtrationseinheiten und den korrespondieren Verfahren, die sich für klassische, mikrobiologische Membrananwendungen bewährt haben, in denen man lediglich Partikel entfernt oder im Bereich der Mikrobiologie entstandene Kolonien visuell auswertet, ist aber, dass nach der Filtration die zurückgehaltenen Partikel oder deren Inhaltsstoffe von der Membran nicht mehr in der Weise durch Abspülen zu Entfernen sind, dass hochkonzentrierte Suspensionen entstehen.

Diese Nachteile sind insbesondere bei Anwendungen von Bedeutung, die nicht dem klassischen Wachstum zurückgehaltener Keime auf Agar entsprechen, sondern die moderne molekularbiologische Markierungs- und Detektionsmethoden einsetzen, gleichgültig, ob es sich um Methoden handelt, die eine Vorinkubation benötigen oder direkt durch Markierungs- oder Verstärkungsmethoden untersucht werden.

Aus der DE 20 2004 005 694 U1 ist ein Gerätesystem zur Flüssigkeitsuntersuchung bekannt, das an einem Rahmen einen Ansaugstutzen aufweist, auf den über einen Adapter ein Filterhalter mit einer Filtrationsmembran bzw. einem Filtrationsmedium befestigt ist, wobei sich auf dem Filterhalter ein abnehmbarer Trichter befindet. Nach einem Filtrationsvorgang wird der Trichter entfernt und zwischen Ansaugstutzen und Adapter wird zur weiteren Untersuchung eine DNS-Bindungssäule eingesteckt.

Nachteilig dabei ist, dass keine universelle Filtrationsvorrichtung bzw. Behandlungsvorrichtung verwendet werden kann. Weiterhin nachteilig ist, dass bei den einzelnen Manipulationen die Gefahr der Kontamination besteht.

Weiterhin nachteilig ist, dass eine reverse Spülung des Filtrationsmediums nicht möglich ist.

Aus der EP 1 566 209 A1 sind eine Vorrichtung und ein Verfahren für eine vakuumunterstützte Affinitätschromatographie bekannt.

Auch hierbei ist eine spezielle Vorrichtung notwendig, bei der zudem durch unterschiedliche austauschbare Komponenten die Gefahr einer Kreuzkontamination besteht. Auch ist bei dieser Vorrichtung, wie bei den anderen Vorrichtungen, eine reversible Durchspülung ohne Entfernung des Filtrationsmediums nicht einfach möglich.

### Aufgabenstellung

Aufgabe der vorliegenden Erfindung ist es daher, eine Vorrichtung und ein Verfahren bereitzustellen, bei dem es möglich ist, ein poröses Filtrationsmedium ohne weitere technische Hilfsmittel einfach und kostengünstig in eine weitere Behandlungsstation einzubringen, in der über die reverse Filtration einer sehr kleinen Flüssigkeitsmenge ein Großteil der zurückgehaltenen Inhaltsstoffe hochkonzentriert in ein kleines Volumen überführt werden kann.

### Darstellung der Erfindung

Die die Vorrichtung betreffende Aufgabe wird in Verbindung mit dem Oberbegriff des Anspruches 1 dadurch gelöst, dass das Aufnahmeteil eine Filtrationsunterstützung aufweist, die auf ihrer dem Filtrationsmedium abgewandten Seite mit einem Auslass für eine reverse Spülung in Verbindung steht. Die dem Filtrationsmedium zugewandte Seite der Filtrationsunterstützung ist der Retentatseite des Filtrationsmediums zugewandt.
Durch die Anordnung einer Filtrationsunterstützung in dem Aufnahmeteil oberhalb des Filtrationsmediums, die mit einem Auslass in Verbindung steht, ist es möglich, die Vorrichtung bzw. das Aufnahmeteil über Kopf in eine weitere Behandlungsstation einzusetzen und eine reverse Spülung durchzuführen, ohne dass das Filtrationsmedium aus dem Aufnahmeteil entnommen werden muss. Das Filtrationsmedium ist bevorzugt scheibenförmig und kann eine poröse Membran oder ein anderer geeigneter Filter sein.
Die Vorrichtung kann eine eigenständige Filtrationsvorrichtung bilden oder zumindest Teil einer eigenständigen Filtrationsvorrichtung sein. Dabei können das Unterteil und das Aufnahmeteil als Einwegartikel ausgebildet sein. Auch kann die Vorrichtung insgesamt als Einwegartikel bzw. aus Einwegartikeln ausgebildet sein.
Das Unterteil kann aber auch Teil einer ersten Filtrationsvorrichtung sein, aus der mit dem Aufnahmeteil das Filtrationsmedium entnommen wird.
Gemäß einer bevorzugten Ausführungsform der Erfindung ist mit dem Auslass des Aufnahmeteils ein lösbares Auffanggefäß verbunden. Dadurch, dass der Auslass bereits mit einem Auffanggefäß verbunden ist, können die partikulären Inhaltsstoffe von dem Filtrationsmedium abgespült und direkt in dem Auffanggefäß aufgefangen werden, ohne das eine zusätzliche Manipulation mit der Gefahr einer Kontamination notwendig ist.

Nach einer weiteren bevorzugten Ausführungsform der Erfindung ist der Fixierrand des Aufnahmeteils mit dem Rand des Filtrationsmediums über eine Klebehaftung verbindbar. Dabei weist der Fixierrand des Aufnahmeteils oder der Rand des Filtrationsmediums eine Haftschicht aus einem geeigneten Kleber auf. Durch die Haftschicht haftet das scheibenförmige Filtrationsmedium an dem Aufnahmeteil und lässt sich leicht und einfach aus der Behandlungsvorrichtung entnehmen und weiter behandeln. Grundsätzlich ist es aber auch möglich, dass der Fixierrand des Aufnahmeteils mit dem Rand des Filtrationsmediums über eine Klemmverbindung verbindbar ist.

Das scheibenförmige Filtrationsmedium, dass auf die vorgesehene Behandlungsvorrichtung abgestimmt ist, ist meist rotationssymmetrisch ausgebildet, kann aber auch eine Rechteck- oder eine andere Vieleckstruktur aufweisen sowie geometrisch unregelmäßige Formate. Die korrespondierende Vorrichtung zur Aufnahme eines Filtrationsmediums bzw. das Aufnahmeteil ist in seiner umlaufenden Kontur funktional auf die Kontur bzw. Struktur des scheibenförmigen Filtrationsmediums so abgestimmt, dass es nur in dem Bereich einen Klebkontakt oder eine Klemmverbindung herstellt, der nicht durch das Medium und die vorangegangene Filtration benetzt wurde und der nicht zur weiteren Analyse verwendet wird.

Die Haftschicht ist aus einem PSA-("Pressure Sensitive Adhesive")-Dispersionsklebstoff oder aus Acrylat-Copolymer-Mikrokugeln ausgebildet. Dadurch sind auch nasse Filter ausreichend am Fixierrand des Aufnahmeteils anhaftbar und ggf. auch wieder abziehbar. Geeignete druckempfindliche Kleber sind dem Fachmann beispielsweise als mikrokugelbasierte Acrylatkleber bekannt. Dabei können Kleber eingesetzt werden, die durch gängige Methoden sterilisierbar sind.

Zudem werden Kleber eingesetzt, die keine unspezifischen Reaktionen oder Signale mit in der nachfolgenden Analytik eingesetzten Reagenzien und Reaktionsmethoden zeigen. Insbesondere ist der Kleber bevorzugt DNA- und proteinfrei und weist keine eluierbaren Substanzen auf, die durch Färbung, Fluoreszenz oder chemische Reaktion die angeschlossene Analytik stören.

Gemäß einer weiteren bevorzugten Ausführungsform der Erfindung ist eine mit dem Aufnahmeteil korrespondierende Abdeckung auf das Aufnahmeteil mit aufgenommenem Filtrationsmedium aufsetzbar, wobei die Abdeckung einen Freiraum aufweist, der als Reservoir und Verteiler für Spülflüssigkeit ausgebildet ist. Damit kann das Aufnahmeteil als ein Flüssigkeitsverteiler eingesetzt werden, der der Kontaktierung, der Dosierung der räumlichen Verteilung und der gleichmäßigen Abgabe der Spülflüssigkeit während des Kontakt- und Abspülvorganges dient. Dabei ist das Aufnahmeteil so ausgebildet, dass es nach Abnahme des porösen Filtrationsmediums mit dem Aufnahmeteil von einer ersten Behandlungsvorrichtung auf die Oberseite bzw. Retentatseite des Filtrationsmediums aufgesetzt und fixiert wird. Dabei kann die Abdeckung die Unterseite des Filtrationsmediums so abschließen, dass eine rückwärtige Kontamination des Filtrationsmediums und der Probevolumina während der Handhabungsschritte ausgeschlossen ist.

Nach einer weiteren bevorzugten Ausführungsform der Erfindung weist die Abdeckung auf der dem Aufnahmeteil abgewandten Seite eine von einem Verschluss mehrfach wiederverschließbare Öffnung auf. Durch die Öffnung können unterschiedliche Mengen und/oder Typen an Spül- oder Behandlungsflüssigkeiten zugegeben werden.

Nach einer weiteren bevorzugten Ausführungsform weist die Abdeckung weitere Öffnungen auf, die so angeordnet und ausgeformt sind, dass eine gleichmäßige, luftblasenfreie Befüllung des Spülflüssigkeitsreservoirs gewährleistet ist.

Gemäß einer weiteren bevorzugten Ausführungsform der Erfindung ist das Reservoir mit einem porösen Medium gefüllt. Das poröse Medium dient zur Aufnahme und Verteilung der Spülflüssigkeit auf dem Filtrationsmedium. Das poröse Medium des Reservoirs kann flächig auf der Unterseite des Filtrationsmediums aufliegen. Die Abdeckung ist mit ihrem Reservoir so ausgebildet, dass sie ein definiertes Volumen aufnehmen und flächig verteilen kann.

Das Aufnahmeteil und die zugehörige Abdeckung können so gestaltet sein, dass das Filtrationsmedium im Bereich außerhalb der Filtrationsfläche durch die Abdeckung radial zum Rand hin abgedichtet wird und dass unter dem Filtrationsmedium liegende Abflusskanäle und -flächen vorgesehen sind, welche mit einem zum Auffanggefäß hin abfallenden Winkel ausgestattet sind. Durch derartige und weitere vergleichbare Abdichtungsmerkmale kann sichergestellt werden, dass im Fall einer Zentrifugationsspülung das komplette Spülmedium durch das Filtrationsmedium in das Auffanggefäß gefördert wird, ohne auf Grund der Zentrifugalkraft seitlich im Bereich außerhalb des Filtrationsmediums zu verbleiben und damit der weiteren Analyse verlorenzugehen.

Die Abdeckung kann steril verpackt und insbesondere an ihrer dem Aufnahmeteil zugewandten Unterseite steril und lösbar durch eine Sterilbarriere verschlossen geliefert werden. Auch ist es möglich, die Abdeckung mit Spülflüssigkeit gefüllt zu liefern. Dabei ist die Spülflüssigkeit besonders geeignet, anhaftende Partikel während der reversen Spülung zu lösen. Insbesondere ist die Spül- bzw. Behandlungsflüssigkeit geeignet, an dem Filtrationsmedium anhaftende Mikroorganismen abzulösen, zu lysieren und/oder die dann freigesetzten Inhaltsstoffe zu extrahieren.

Durch den Kontakt der mit Behandlungsflüssigkeit gefüllten Abdeckung kann über einen entsprechenden Zeit- und Temperaturverlauf sichergestellt werden, dass entsprechende Reaktionen mit den anhaftenden Keimen abgeschlossen werden, bevor durch die reverse Spülung die Reaktionsprodukte in das Auffanggefäß oder in anschließende Filter- oder Adsorptionseinheiten überführt werden.

Das Aufnahmeteil und die Abdeckung sind für die entsprechende nachfolgende Analytik geeignet und insbesondere frei von störenden Kontaminationen. Insbesondere für die PCR sind DNAfreie Produkte notwendig.

Nach einer weiteren bevorzugten Ausführungsform der Erfindung ist zwischen dem Auslass des Aufnahmeteils und dem Auffanggefäß ein entnehmbarer Filter und/oder eine entsprechende Adsorptionseinheit eingefügt, die weitere Behandlungsschritte zur Konzentrierung, Reinigung und Elution erlauben.

Die Aufgabe betreffend das Verfahren wird in Verbindung mit dem Oberbegriff des Anspruches 11 dadurch gelöst, dass das Aufnahmeteil mit dem aufgenommene Filtrationsmedium über Kopf gewendet wird und dass an dem Filtrationsmedium zurückgehaltene Inhaltsstoffe revers abgespült und in einem lösbar verbundenen Auffanggefäß aufgefangen werden.

Durch das Wenden des Aufnahmeteils mit dem aufgenommenen Filtrationsmedium über Kopf, ist es nicht notwendig, das Filtrationsmedium für eine reverse Spülung aus dem Aufnahmeteil heraus zu nehmen. Durch das mit dem Aufnahmeteil verbundene Auffanggefäß wird zudem zuverlässig verhindert, dass es beim Aufsetzen des Auffanggefäßes zu unerwünschten Kontaminationen kommen kann. Der Aufwand für eine reverse Spülung wird so erheblich vereinfacht und sicherer gestaltet.

Die Vorrichtung zur Behandlung des Filtrationsmediums kann steril verpackt geliefert werden. Dabei kann das Aufnahmeteil an seiner Aufnahmeöffnung, die an der dem Auslass abgewandten Unterseite angeordnet ist, von einem Sterilverschluss verschlossen sein, der beispielsweise als eine anhaftende Folie ausgebildet ist. Mit einer Lasche ragt der Sterilverschluss seitlich über den Umfang des Aufnahmeteils hinaus.

Vor dem ersten Einsatz der Vorrichtung bzw. des Aufnahmeteils wird der Sterilverschluss an der Lasche erfasst und von dem Aufnahmeteil abgezogen.

Gemäß einer bevorzugten Ausführungsform der Erfindung wird nach dem Wenden des Aufnahmeteiles eine Abdeckung mit einem ein Reservoir bildenden Freiraum, der mit Spülflüssigkeit gefüllt wurde, auf das Aufnahmeteil aufgesetzt und in eine weitere Behandlungsvorrichtung eingebracht, wobei die Flüssigkeit aus dem Reservoir zum Abspülen der Inhaltsstoffe abgegeben wird.

Durch das Aufsetzen der Abdeckung auf das Aufnahmeteil nach dessen Wenden, kann das Aufnahmeteil mit dem Filtrationsmedium und der Abdeckung einfach und sicher in eine weitere Behandlungsstation, z.B. eine Zentrifuge, eingebracht werden. Ohne zusätzliche Manipulationen kann in der Behandlungsstation das Filtrationsmedium revers mit der Flüssigkeit aus dem Reservoir gespült respektive zentrifugiert werden, so dass die zurückgehaltenen vorzugsweise partikulären Inhaltsstoffe abgespült und von dem Auffanggefäß des Aufnahmeteils aufgefangen werden.

Die Abdeckung kann ebenfalls steril verpackt geliefert werden. Insbesondere kann der als Spülflüssigkeitsverteiler ausgebildete Freiraum der Abdeckung zum Aufnahmeteil hin ebenfalls steril und lösbar durch eine Sterilbarriere verschlossen geliefert werden. Die Sterilbarriere kann beispielsweise als eine anhaftende Folie ausgebildet sein.

Mit einer Lasche ragt die Sterilbarriere seitlich über den Umfang der Abdeckung hinaus.

Vor dem ersten Einsatz der Abdeckung wird die Sterilbarriere an der Lasche erfasst und von der Abdeckung abgezogen.

Damit ergibt sich ein einfaches und kostengünstiges Manipulieren des Filtrationsmediums mit der aufgebrachten Probe.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibungen der Zeichnungen

In den Zeichnungen zeigen:
- Figur 1:: Eine Seitenansicht einer Vorrichtung zur Behandlung eines Filtrationsmediums im Schnitt mit einem Aufnahmeteil mit einer Filtrationsunterstützung, einem Auslass mit aufgestecktem Auffanggefäß und einem Sterilverschluss,
- Figur 2:: eine Seitenansicht im Schnitt einer ersten Filtrationsvorrichtung mit einem auf einem Unterteil angeordneten Filtrationsmedium,
- Figur 3:: eine Seitenansicht im Schnitt des Aufnahmeteils von Figur 1 auf dem Unterteil mit Filtrationsmedium von Figur 2,
- Figur 4:: eine Seitenansicht im Schnitt des über Kopf gewendeten Aufnahmeteils von Figur 1 mit Filtrationsmedium und aufgesetzter Abdeckung,

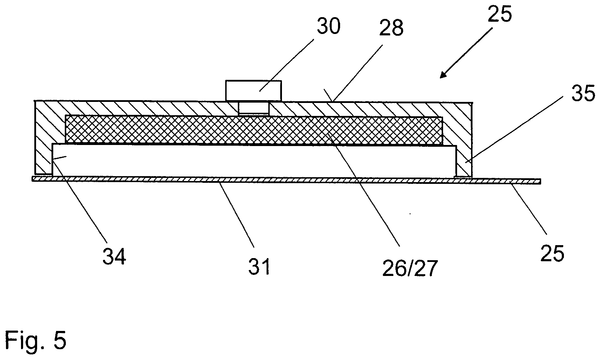

- Figur 5:: eine Seitenansicht im Schnitt der Abdeckung von Figur 4 mit einer Sterilbarriere,
- Figur 6:: eine Seitenansicht im Schnitt eines weiteren Aufnahmeteils mit Filtrationsmedium und aufgesetzter Abdeckung und
- Figur 7:: eine Seitenansicht im Schnitt einer weiteren Vorrichtung zur Behandlung eines Filtrationsmediums im Schnitt, bestehend aus einem Aufnahmeteil mit einer Filtrationsunterstützung, einem Auslass mit aufgestecktem Auffanggefäß und einem Sterilverschluss.

### Beschreibung der Ausführungsbeispiele

Eine Vorrichtung 1 besteht im Wesentlichen aus einem Aufnahmeteil 2, einer Filtrationsunterstützung 3 und einem Auslass 4.

Das Aufnahmeteil 2 bildet eine umlaufende Kontur mit einer Außenwandung 5 und einer parallel verlaufenden Innenwandung 6, die einen Absatz 7 im Aufnahmeteil 2 umschließt, der in vertikaler Richtung nach oben von einer Deckenwandung 8 begrenzt wird, die eine zentrale Öffnung 9 in dem an ihr angeformten zylindrischen Auslass 4 aufweist. In dem Absatz 7 ist die Filtrationsunterstützung 3 angeordnet, die aus einem flüssigkeitsdurchlässigen Material besteht.

Das Aufnahmeteil 2 weist an seiner der Deckenwandung 8 abgewandten Aufnahmeteilinnenfläche 10 die Innenwandung 6 auf, deren freies Ende mit seiner Stirnfläche einen Fixierrand 11 bildet. In den Ausführungsbeispielen weist der Fixierrand 11 eine Haftschicht 12 aus einem geeigneten Klebestoff auf.

Die Haftschicht 12 ist beispielsweise aus einem PSA-Dispersionsklebstoff oder aus Acrylat-Copolymer-Mikrokugeln ausgebildet.

Auf die Mantelfläche 13 des Auslasses 4 ist ein Auffanggefäß 14 aufgesteckt. Das mit dem Auslass 4 lösbar verbundene Auffanggefäß 14 ist beispielsweise als ein an sich bekanntes Eppendorf-Gefäß oder "Spin Tube" ausgebildet.

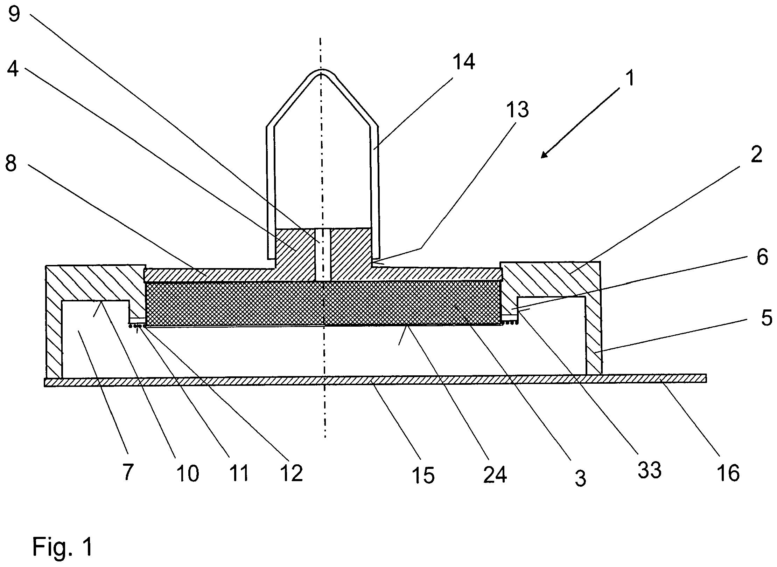

Entsprechend dem Ausführungsbeispiel der Figur 1 weist das Aufnahmeteil 2 an seinem freien Ende der Außenwandung 5 einen versiegelnden Sterilverschluss 15 auf, der über eine Lasche 16 von dem Aufnahmeteil 2 bzw. der Außenwandung 5 abziehbar ist.

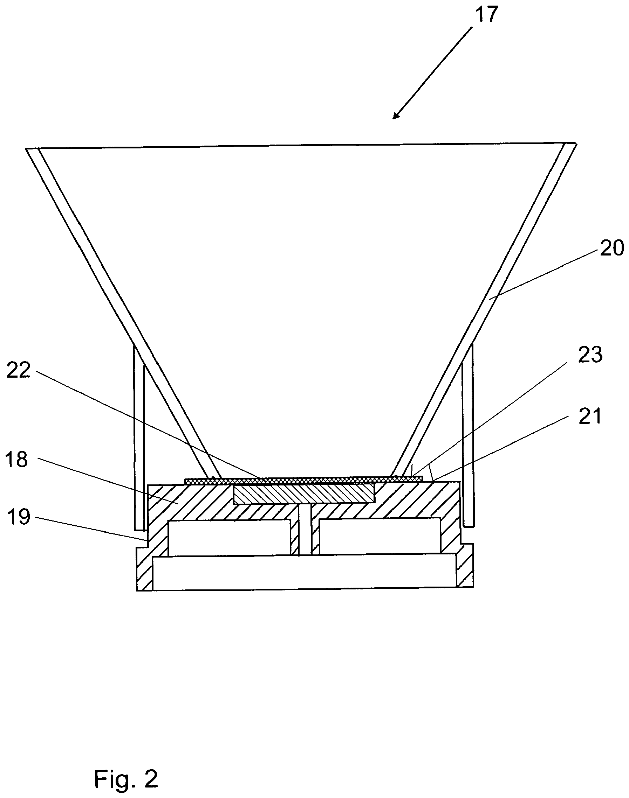

Eine an sich bekannte erste Behandlungsvorrichtung, die entsprechend Figur 2 als eine Filtrationsvorrichtung 17 ausgebildet ist, besteht aus einem Unterteil 18 mit einem Aufnahmeabsatz 19, auf dem ein trichterförmiger Aufsatz 20 aufsetzbar ist. Zwischen dem Aufsatz 20 und einer Filterunterstützungsfläche 21 des Unterteils 18 ist ein bevorzugt scheibenförmiges Filtrationsmedium 22, das beispielsweise als eine poröse Filtermembran ausgebildet ist, angeordnet.

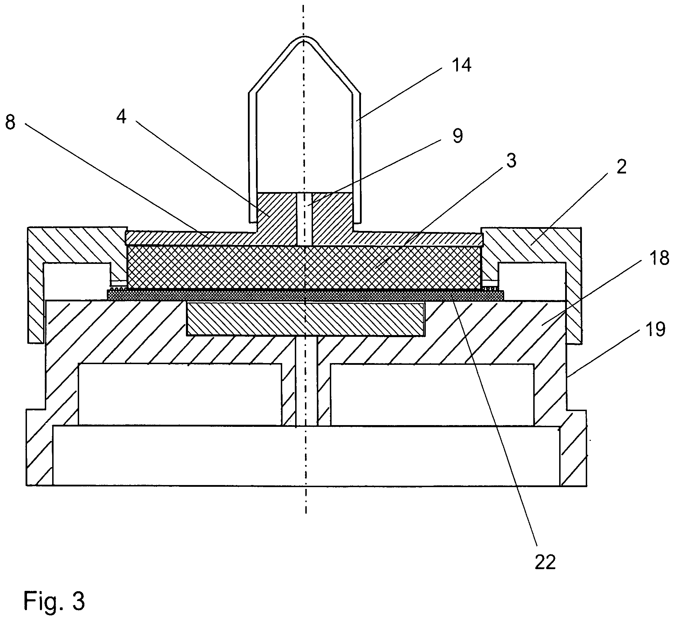

Nach einem Filtrationsvorgang kann der Aufsatz 20 von dem Unterteil 18 abgenommen werden und das Aufnahmeteil 2 der Vorrichtung 1 kann mit aufgestecktem Auffanggefäß 14 anstelle des Aufsatzes 20 auf das Unterteil 18 aufgesetzt werden. Dabei wird das Aufnahmeteil 2 mit seinem Fixierrand 11 auf einen Rand 23 des scheibenförmigen Filtrationsmediums 22 aufgesetzt, so dass das scheibenförmige Filtrationsmedium 22 an der Haftschicht 12 des Fixierrandes 11 haftet und von dem Unterteil 18 gemäß Figur 3 aufgenommen werden kann.

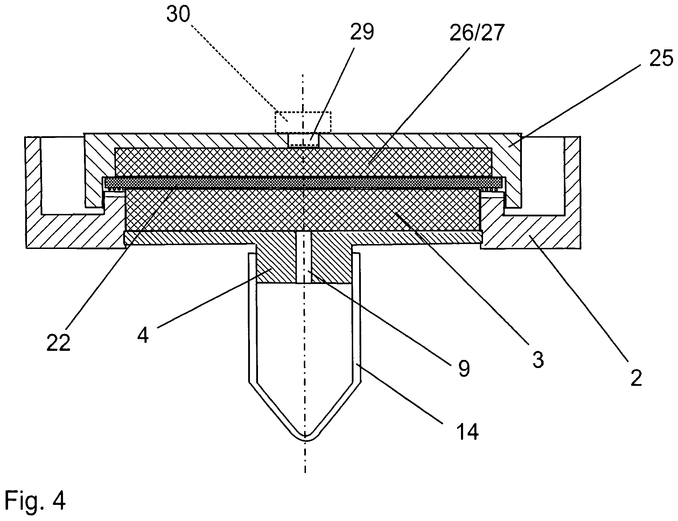

Die Vorrichtung 1 bzw. das Aufnahmeteil 2 kann nunmehr entsprechend Figur 4 mit dem Filtrationsmedium 22 über Kopf gewendet werden, wobei das scheibenförmige poröse Filtrationsmedium 22 auf der der Deckenwandung 8 abgewandten Unterseite 24 der Filtrationsunterstützung aufliegt.

Auf die Innenwandung 6 des Aufnahmeteils 2 mit dem Filtrationsmedium 22 kann zum Verschluss der Vorrichtung 1 bzw. des Aufnahmeteils 2 entsprechend Figur 4 eine Abdeckung 25 aufgesetzt werden.

Die Abdeckung 25 beinhaltet einen Freiraum 26, der als Reservoir und Verteiler für Spülflüssigkeit dient. Der Freiraum 26 ist mit einem porösem Medium 27 ausgefüllt, das Spülflüssigkeit speichern kann und als Verteiler dient.

Die Abmessung der Abdeckung 25 ist so geschaffen, dass sie den Fixierrand 11 des Aufnahmeteils 2 an der Mantelfläche 33 der Innenwandung 6 seitlich mit ihrer Innenfläche 34 der Seitenwandung 35 umfasst und auf dem Filtrationsmedium 22 zu liegen kommt.

Die Abdeckung 25 weist an ihrer dem Aufnahmeteil 2 abgewandten Außenseite 28 eine zentrale Öffnung 29 für Spülschritte auf, die von einem Verschluss 30 verschließbar ist. Die Öffnung 29 erlaubt ein gleichmäßiges, flächiges Benetzen und Belüften des Reservoirs bzw. Freiraumes 26 in dessen poröses Medium 27, welches sich leicht mit Spülflüssigkeit tränken lässt und diese während des Spülvorgangs z.B. durch Zentrifugation auch wieder gleichmäßig abgibt.

Für den Spülvorgang muss zwischen dem Auffanggefäß 14 und dem Auslass 4, sowie dem Verschluss 30 und der Abdeckung 25 eine Druckausgleichseinrichtung, z. B. eine Luftöffnung, bestehen, um die Flüssigkeitsveränderungen entsprechend bewerkstelligen zu können.

Der Spülvorgang kann durch unterschiedliche treibende Kräfte durchgeführt werden, z.B. durch Vakuumfiltration nach Öffnen des Verschlusses 30 und des dichtenden Aufsetzens der Aufnahmeteils 2.

Die Zentrifugation wird bevorzugt, da hierbei kleinste Volumina im Auffanggefäß 14 gesammelt werden können.

Die Abdeckung 25 mit dem als Spülflüssigkeitsreservoir ausgebildeten Freiraum 26 kann auch steril mit einer Sterilbarriere 31 inkl. Öffnungslasche 32 angeboten werden, um eine Kontamination der Probe zu vermeiden.

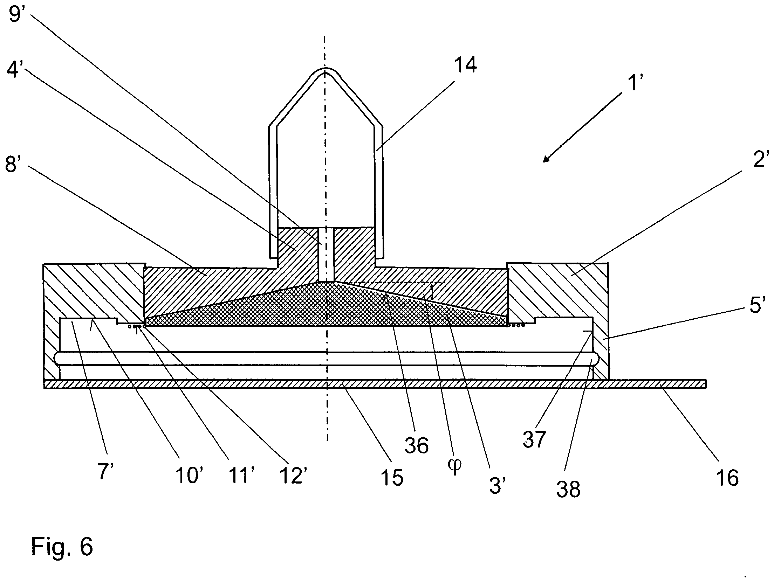

In Figur 6 ist eine Vorrichtung 1' dargestellt, deren Aufnahmeteil 2' mit der Filtrationsunterstützung 3' speziell für eine Zentrifugationsanwendung ausgeführt ist. Das Aufnahmeteil 2' bildet eine umlaufende Kontur mit einer Außenwandung 5', die einen Absatz 7' im Aufnahmeteil 2' umschließt, der in vertikaler Richtung nach oben von einer Deckenwandung 8' begrenzt wird, die eine zentrale Öffnung 9' in dem an ihr angeformten zylindrischen Auslass 4' aufweist. In dem Absatz 7' ist die Filtrationsunterstützung 3' angeordnet, die aus einem flüssigkeitsdurchlässigen Material besteht.

Das Aufnahmeteil 2' weist an seiner der Deckenwandung 8' abgewandten Aufnahmeteilinnenfläche 10' einen kreisförmigen Fixierrand 11' auf, der die Filtrationsunterstützung 3' umschließt. In den Ausführungsbeispielen weist der Fixierrand 11, 11' eine Haftschicht 12 aus einem geeigneten Klebestoff auf. Die Deckenwandung 8' ist so ausgeführt, dass ihre innenliegende Abflussfläche 36 gegenüber einer Horizontalen mit einem zum Auffanggefäß 14 hin abfallenden Winkel ϕ kegelstumpfförmig ausgebildet ist.

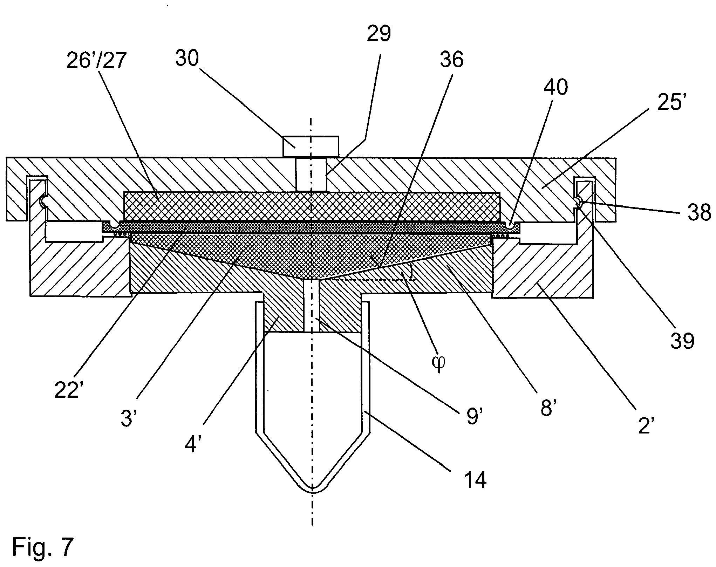

In das Aufnahmeteil 2' mit dem Filtrationsmedium 22 kann zum Verschluss der Vorrichtung 1' bzw. des Aufnahmeteils 2' entsprechend Figur 7 eine Abdeckung 25' aufgesetzt werden.

Die Abdeckung 25' beinhaltet einen Freiraum 26', der als Reservoir und Verteiler für Spülflüssigkeit dient. Der Freiraum 26' ist mit einem porösem Medium 27 ausgefüllt, das Spülflüssigkeit speichern kann und als Verteiler dient.

Die Abmessung der Abdeckung 25' ist so geschaffen, dass sie den Fixierrand 11' des Aufnahmeteils 2' und das Filtrationsmedium 22' komplett überdeckt, auf dem Filtrationsmedium 22' zu liegen kommt und den Innenraum des Aufnahmeteils 2' ausfüllt.

Die Abdeckung 25' weist an ihrer dem Aufnahmeteil 2' abgewandten Außenseite 28' eine zentrale Öffnung 29' für Spülschritte auf, die von einem Verschluss 30' verschließbar ist. Die Öffnung 29' erlaubt ein gleichmäßiges, flächiges Benetzen und Belüften des Reservoirs bzw. Freiraumes 26' in dessen poröses Medium 27, welches sich leicht mit Spülflüssigkeit tränken lässt und diese während des Spülvorgangs z.B. durch Zentrifugation auch wieder gleichmäßig abgibt.

In der Innenfläche 37 der Außenwandung 5' ist eine umlaufende ringförmige Vertiefung 38angeordnet, die mit einer Wulst 39 der Abdeckung 25' korrespondiert. Damit wird die Abdeckung 25' fixiert. Das Filtrationsmedium 22 wird radial nach außen mit einem Dichtrand 40 der Abdeckung 25' abgedichtet, indem der Dichtrand 40 gemäß Figur 7 in das Filtrationsmedium 22 presst bzw. drückt.

Durch derartige und weitere vergleichbare Abdichtungseinrichtungen wird sichergestellt, dass im Fall einer Zentrifugationsspülung das komplette Spülmedium durch das Filtrationsmedium 22' in das Auffanggefäß gefördert wird, ohne auf Grund der Zentrifugalkraft seitlich im Bereich außerhalb des Filtrationsmediums 22' zu verbleiben und damit der weiteren Analyse verloren zu gehen.

## Patentansprüche

1. Vorrichtung (1, 1')zur Behandlung eines porösen Filtrationsmediums (22, 22') mit einem Aufnahmeteil (2, 2'), das auf ein Unterteil (18) mit dem porösen Filtrationsmedium (22. 22') aufsetzbar ist, wobei eine Außenwandung (5, 5') des Aufnahmeteils (2, 2') außerhalb einer für eine Filtration nutzbaren Fläche des Filtrationsmediums (22, 22') positionierbar ist und ein in dem Aufnahmeteil (2, 2') angeordneter Fixierrand (11, 11') auf einem Rand (23) des Filtrationsmediums (22, 22') positionierbar ist,
**dadurch gekennzeichnet,**
**dass** das Aufnahmeteil (2, 2') eine Filtrationsunterstützung (3) aufweist, die auf ihrer dem Filtrationsmedium (22, 22') abgewandten Seite mit einem Auslass (4) für eine reverse Spülung in Verbindung steht und die auf ihrer dem Filtrationsmedium (22, 22') zugewandten Seite der Retentatseite des Filtrationsmediums (22, 22') zugewandt ist.

2. Vorrichtung nach Anspruch 1,
**dadurch gekennzeichnet,**
**dass** das Unterteil (18) Teil einer ersten Filtrationsvorrichtung (17) ist, aus der mit dem Aufnahmeteil (2) das Filtrationsmedium (22) entnehmbar ist.

3. Vorrichtung nach Anspruch 1 oder 2,
**dadurch gekennzeichnet,**
**dass** mit dem Auslass (4) ein lösbares Auffanggefäß (14) verbunden ist.

4. Vorrichtung nach einem der Ansprüche 1 bis 3,
**dadurch gekennzeichnet,**
**dass** der Fixierrand (11, 11') des Aufnahmeteils (2, 2') mit dem Rand (23) des Filtrationsmediums (22, 22') über eine Klebehaftung verbindbar ist.

5. Vorrichtung nach einem der Ansprüche 1 bis 3,
**dadurch gekennzeichnet,**
**dass** der Fixierrand (11, 11') des Aufnahmeteils (2) mit dem Rand (23) des Filtrationsmediums (22, 23') über eine Klemmverbindung verbindbar ist.

6. Vorrichtung nach einem der Ansprüche 1 bis 4,
**dadurch gekennzeichnet,**
**dass** der Fixierrand (11, 11') des Aufnahmeteils (2, 2') oder der Rand (23) des Filtrationsmediums (22, 22') eine Haftschicht (12) aus einem geeigneten Kleber aufweist.

7. Vorrichtung nach einem der Ansprüche 1 bis 6,
**dadurch gekennzeichnet,**
**dass** eine mit dem Aufnahmeteil (2, 2') korrespondierende Abdeckung (25, 25') auf das Aufnahmeteil (2, 2') mit Filtrationsmedium (22, 22') aufsetzbar ist und
**dass** die Abdeckung (25, 25') einen Freiraum (26, 26') aufweist, der als Reservoir und Verteiler für Spülflüssigkeit ausgebildet ist.

8. Vorrichtung nach Anspruch 7,
**dadurch gekennzeichnet,**
**dass** die Abdeckung (25, 25') auf ihrer dem Aufnahmeteil (2, 2') abgewandten Seite eine von einem Verschluss (30) verschließbare Öffnung (29) aufweist.

9. Vorrichtung nach einem der Ansprüche 1 bis 5,
**dadurch gekennzeichnet,**
**dass** zwischen dem Auslass (4, 4') und dem Auffanggefäß (14) ein entnehmbarer Filter und/oder eine entsprechende Adsorptionseinheit eingefügt sind, die weitere Behandlungsschritte zur Konzentrierung, Reinigung und Elution erlauben.

10. Vorrichtung (1, 1') nach einem der Ansprüche 1 bis 9,
**dadurch gekennzeichnet,**
**dass** das Unterteil (18) und das Aufnahmeteil (2, 2') Einwegartikel sind.

11. Verfahren zur Behandlung eines porösen Filtrationsmediums (22, 22'), bei dem ein Aufnahmeteil (2, 2') einer Vorrichtung (1, 1') auf das in einem Unterteil (18) einer Filtrationsvorrichtung (17) angeordnete und einer flüssigen Probe ausgesetzte Filtrationsmedium (22) aufgesetzt wird, wobei ein in dem Aufnahmeteil (2, 2') angeordneter Fixierrand (11, 11') mit einem Rand (23) des Filtrationsmediums (22, 22') verbunden und mit dem verbundenen Filtrationsmedium (22, 22') von dem ersten Unterteil (18) abgehoben wird,
**dadurch gekennzeichnet,**
**dass** das Aufnahmeteil (2, 2') mit dem aufgenommenen Filtrationsmedium (22, 22') über Kopf gewendet wird, wobei das scheibenförmige poröse Filtrationsmedium (22, 22') auf einer einer Deckenwandung (8) abgewandten Unterseite (24) einer Filtrationsunterstützung (3) aufliegt, und
**dass** an dem Filtrationsmedium (22, 22') zurückgehaltene Inhaltsstoffe revers abgespült und in einem lösbar verbundenen Auffanggefäß (14)aufgefangen werden.

12. Verfahren nach Anspruch 11,
**dadurch gekennzeichnet,**
**dass** nach dem Wenden des Aufnahmeteiles (2, 2') eine Abdeckung (25, 25') mit einem ein Reservoir bildenden Freiraum (26, 26'), der mit Spülflüssigkeit gefüllt wurde, auf das Aufnahmeteil (2, 2') aufgesetzt und in eine weitere Behandlungsvorrichtung eingebracht wird, wobei die Flüssigkeit aus dem Freiraum (26, 26') zum Abspülen der Inhaltsstoffe abgegeben wird.

## Claims

1. Device (1, 1') for treating a porous filtration medium (22, 22'), comprising a receiving part (2, 2') placeable on a lower part (18) with the porous filtration medium (22, 22'), wherein an outer wall (5, 5') of the receiving part (2, 2') is positionable outside an area, which is usable for a filtration, of the filtration medium (22, 22') and a fixing edge (11, 11'), which is arranged in the receiving part (2, 2'), is positionable on an edge (23) of the filtration medium (22, 22'), **characterised in that** the receiving part (2, 2') comprises a filtration support (3) which at its side remote from the filtration medium (22, 22') is connected with an outlet (4) for reverse flushing and at its side facing the filtration medium (22, 22') faces the retentate side of the filtration medium (22, 22').

2. Device according to claim 1, **characterised in that** the lower part (18) is part of a first filtration device (17) from which the filtration medium (22) is removable together with the receiving part (2).

3. Device according to claim 1 or 2, **characterised in that** a detachable collection vessel (14) is connected with the outlet (4).

4. Device according to any one of claims 1 to 3, **characterised in that** the fixing edge (11, 11') of the receiving part (2, 2') is connectible with the edge (23) of the filtration medium (22, 22') by way of an adhesive connection.

5. Device according to any one of claims 1 to 3, **characterised in that** the fixing edge (11, 11') of the receiving part (2) is connectible with the edge (23) of the filtration medium (22, 23') by way of a clamping connection.

6. Device according to any one of claims 1 to 4, **characterised in that** the fixing edge (11, 11') of the receiving part (2, 2') or the edge (23) of the filtration medium (22, 22') has an adhesive layer (12) of a suitable adhesive.

7. Device according to any one of claims 1 to 6, **characterised in that** a cover (25, 25') co-operating with the receiving part (2, 2') is placeable on the receiving part (2, 2') with the filtration medium (22, 22') and that the cover (25, 25') has a free space (26, 26') constructed as a reservoir and distributor for flushing liquid.

8. Device according to claim 7, **characterised in that** the cover (25, 25') has on its side remote from the receiving part (2, 2') an opening (29) closable by a closure (30).

9. Device according to any one of claims 1 to 5, **characterised in that** a removable filter and/or a corresponding adsorption unit, which allows or allow further handling steps for concentrating, cleaning and elution, is or are introduced between the outlet (4, 4') and the collection vessel (14).

10. Device (1, 1') according to any one of claims 1 to 9, **characterised in that** the lower part (18) and the receiving part (2, 2') are single-use articles.

11. Method for treating a porous filtration medium (22, 22'), in which a receiving part (2, 2') of a device (1, 1') is placed on the filtration medium (22), which is arranged in a lower part (18) of a filtration device (17) and which is exposed to a liquid sample, wherein a fixing edge (11, 11') arranged in the receiving part (2, 2') is connected with an edge (23) of the filtration medium (22, 22') and raised together with the connected filtration medium (22, 22') from the first lower part (18), **characterised in that** the receiving part (2, 2') with the received filtration medium (22, 22') is inverted, wherein the disc-shaped porous filtration medium (22, 22') rests on a lower side (24), which is remote from a cover wall (8), of a filtration support (3) and that constituents restrained at the filtration medium (22, 22') are flushed out in reverse and collected in a detachably connected collection vessel (14).

12. Method according to claim 11, **characterised in that** after turning over the receiving part (2, 2') a cover (25, 25') with a free space (26, 26'), which forms a reservoir and which was filled with flushing liquid, is placed on the receiving part (2, 2') and introduced into a further treatment device, wherein the liquid is delivered from the free space (26, 26') for flushing out the constituents.

## Revendications

1. Dispositif (1, 1') de traitement d'un milieu de filtration (22, 22') poreux, comprenant une partie de réception (2, 2') qui peut être posée sur une partie inférieure (18) contenant le milieu de filtration (22, 22') poreux, dans lequel une paroi extérieure (5, 5') de la partie de réception (2, 2') peut être positionnée à l'extérieur d'une surface du milieu de filtration (22, 22') pouvant être utilisée pour une filtration, et un bord de fixation (11, 11') agencé dans la partie de réception (2, 2') peut être positionné sur un bord (23) du milieu de filtration (22, 22'),
**caractérisé**
**en ce que** la partie de réception (2, 2') comprend un support de filtration (3), qui est relié sur sa face opposée au milieu de filtration (22, 22') à une sortie (4) aux fins de rinçage inverse et qui, sur sa face tournée vers le milieu de filtration (22, 22'), est tourné vers le côté rétentat du milieu de filtration (22, 22').

2. Dispositif selon la revendication 1,
**caractérisé**
**en ce que** la partie inférieure (18) fait partie d'un premier dispositif de filtration (17) dont le milieu de filtration (22) peut être retiré en même temps que la partie de réception (2).

3. Dispositif selon la revendication 1 ou 2,
**caractérisé**
**en ce qu'**un récipient collecteur (14) amovible est relié à la sortie (4).

4. Dispositif selon l'une quelconque des revendications 1 à 3,
**caractérisé**
**en ce que** le bord de fixation (11, 11') de la partie de réception (2, 2') peut être relié au bord (23) du milieu de filtration (22, 22') au moyen d'une liaison adhésive.

5. Dispositif selon l'une quelconque des revendications 1 à 3,
**caractérisé**
**en ce que** le bord de fixation (11, 11') de la partie de réception (2) peut être relié au bord (23) du milieu de filtration (22, 23') au moyen d'une liaison par serrage.

6. Dispositif selon l'une quelconque des revendications 1 à 4,
**caractérisé**
**en ce que** le bord de fixation (11, 11') de la partie de réception (2, 2') ou le bord (23) du milieu de filtration (22, 22') présente une couche adhésive (12) composée d'une colle adaptée.

7. Dispositif selon l'une quelconque des revendications 1 à 6,
**caractérisé**
**en ce qu'**un couvercle (25, 25') correspondant à la partie de réception (2, 2') peut être posé sur la partie de réception (2, 2') contenant le milieu de filtration (22, 22') et en ce que le couvercle (25, 25') présente un espace libre (26, 26') qui est réalisé sous la forme d'un réservoir et d'un distributeur de liquide de rinçage.

8. Dispositif selon la revendication 7,
**caractérisé**
**en ce que** le couvercle (25, 25') présente sur sa face opposée à la partie de réception (2, 2') une ouverture (29) pouvant être fermée par une fermeture (30).

9. Dispositif selon l'une quelconque des revendications 1 à 5,
**caractérisé**
**en ce qu'**un filtre amovible et/ou une unité d'adsorption correspondante, qui permettent d'autres étapes de traitement aux fins de concentration, de nettoyage et d'élution sont insérés entre la sortie (4, 4') et le récipient collecteur (14).

10. Dispositif (1, 1') selon l'une quelconque des revendications 1 à 9,
**caractérisé**
**en ce que** la partie inférieure (18) et la partie de réception (2, 2') sont des articles jetables.

11. Procédé de traitement d'un milieu de filtration (22, 22') poreux, selon lequel une partie de réception (2, 2') d'un dispositif (1, 1') est posée sur le milieu de filtration (22) agencé dans une partie inférieure (18) d'un dispositif de filtration (17) et exposé à un échantillon liquide, dans lequel un bord de fixation (11, 11') agencé dans la partie de réception (2, 2') est relié à un bord (23) du milieu de filtration (22, 22') et soulevé, tout en étant relié au milieu de filtration (22, 22'), de la première partie inférieure (18),
**caractérisé**
**en ce que** la partie de réception (2, 2') dans laquelle est reçu le milieu de filtration (22, 22') est retournée de manière à être tête en bas, dans lequel le milieu de filtration (22, 22') poreux en forme de disque repose sur une face inférieure (24) d'un support de filtration (3) opposée à une paroi supérieure (8), et en ce que les substances contenues retenues sur le milieu de filtration (22, 22') subissent un rinçage inverse et sont collectées dans un récipient collecteur (14) relié de manière libérable.

12. Procédé selon la revendication 11,
**caractérisé**
**en ce que**, une fois la partie de réception (2, 2') retournée, un couvercle (25, 25') comprenant un espace libre (26, 26') formant un réservoir, lequel espace a été rempli de liquide de rinçage, est posé sur la partie de réception (2, 2') et est introduit dans un autre dispositif de traitement, dans lequel le liquide est distribué depuis l'espace libre (26, 26') pour le rinçage des substances contenues.
